# FASCICULE DE BREVET EUROPEEN

(11) **EP 3 583 411 B1**
(45) Date de publication et mention de la délivrance du brevet: **14.04.2021**
(21) Numéro de dépôt: 18703069.7
(22) Date de dépôt: 18.01.2018
(51) Int. Cl.: G01N 25/52, G01N 33/28

(54) **PROCÉDÉ DE DÉTERMINATION DE LA PROPENSION D'UNE HUILE MOTEUR A CONDUIRE A UN PRE-ALLUMAGE D'UN MOTEUR DE VÉHICULE**
VERFAHREN ZUR BESTIMMUNG DER NEIGUNG EINES MOTORENÖLS ZUR VORZÜNDUNG EINES KRAFTFAHRZEUGMOTORS
METHOD FOR DETERMINING THE PROPENSITY OF AN ENGINE OIL TO LEAD TO PRE-IGNITION OF A VEHICLE ENGINE

(30) Priorité: 14.02.2017 FR 1751186
(43) Date de publication de la demande: 25.12.2019
(73) Titulaire: PSA Automobiles SA, 78300 Poissy (FR)
(72) Inventeur: CREPEAU, Gerald, 92700 Colombes (FR); LODE, Christophe, 25260 Etouvans (FR)
(86) Numéro de dépôt international: PCT/FR2018/050128
(87) Numéro de publication internationale: WO 2018/150113

(56) Documents cités:
- KOSUKE FUJIMOTO ET AL: "Engine Oil Development for Preventing Pre-Ignition in Turbocharged Gasoline Engine", SAE INTERNATIONAL JOURNAL OF FUELS AND LUBRICANTS, vol. 7, no. 3, 15 avril 2014 (2014-04-15), pages 869-874, XP055248491, US ISSN: 1946-3960, DOI: 10.4271/2014-01-2785
- A. B. VIPPER ET AL: "Engine oil ageing under laboratory conditions", LUBRICATION SCIENCE, vol. 14, no. 3, 1 mai 2002 (2002-05-01), pages 363-375, XP055406677, US ISSN: 0954-0075, DOI: 10.1002/ls.3010140307
- ORIAN WELLING ET AL: "Impact of Lubricant Composition on Low-speed Pre-Ignition", SAE TECHNICAL PAPER SERIES, vol. 2014-01-1213, 1 avril 2014 (2014-04-01), page 5 pp., XP055203773, US ISSN: 0148-7191, DOI: 10.4271/2014-01-1213
- KAZUO TAKEUCHI ET AL: "Investigation of Engine Oil Effect on Abnormal Combustion in Turbocharged Direct Injection - Spark Ignition Engines", SAE INTERNATIONAL JOURNAL OF FUELS AND LUBRICANTS, vol. 5, no. 3, 30 janvier 2012 (2012-01-30), pages 1017-1024, XP055203823, ISSN: 1946-3960, DOI: 10.4271/2012-01-1615

## Description

L'invention concerne, de façon générale, le domaine du pré-allumage d'un moteur thermique, notamment installé dans un véhicule automobile.

L'invention porte plus précisément sur un procédé de détermination de la propension d'une huile moteur à conduire à un pré-allumage d'un moteur de véhicule automobile, lors de son vieillissement.

De manière connue, le moteur thermique d'un véhicule, également connu sous le nom de moteur à combustion interne, comprend des cylindres creux, désignés chambres de combustion, dans lesquels est injecté un mélange d'air et de carburant. Un tel mélange est compressé par un piston coulissant dans chaque chambre de combustion. La détente du mélange génère la poussée du piston, entraînant la rotation d'un arbre moteur, entraînant à son tour la rotation des roues du véhicule. Ainsi, le moteur à combustion interne permet de transformer l'énergie thermique dégagée par la combustion du mélange en énergie mécanique entraînant la mise en mouvement du véhicule.

Dans le cas d'un moteur à essence, une bougie est montée dans le moteur de manière à générer une étincelle dans la chambre de combustion afin de déclencher la combustion du mélange d'air et de carburant à un moment prédéterminé permettant d'optimiser le fonctionnement du moteur.

Cependant, de manière connue, il arrive que la combustion soit déclenchée avant le moment prédéterminé, on parle alors de pré-allumage du mélange dans la chambre de combustion. Le phénomène de pré-allumage se produit notamment lorsque la vitesse de rotation du moteur est faible, un tel phénomène est désigné pré-allumage à vitesse réduite ou LSPI pour Low Speed Pre-Ignition en langue anglaise. Le pré-allumage à vitesse réduite affecte les moteurs fonctionnant à basse vitesse moteur (faible nombre de tours par minute de l'arbre moteur) et à charges élevées, la charge d'un moteur étant le rapport entre le travail fourni par le moteur à une certaine vitesse de rotation du moteur et le travail maximal que le moteur peut fournir à cette vitesse. A titre d'exemple, une charge élevée est représentative d'un véhicule se déplaçant sur une forte pente ou bien d'un véhicule chargé d'une remorque ou d'une caravane.

De manière connue, le pré-allumage à vitesse réduite se produit par exemple en cas de niveaux élevés de vapeur d'huile ou de présence de points chauds dans la chambre de combustion et peut également être dû à la présence de gouttelettes d'huile moteur pendant la phase de compression du mélange air/carburant précédant l'étincelle d'allumage. Un tel phénomène de pré-allumage peut dégrader le moteur en entraînant une augmentation brusque de la température dans la chambre de combustion ainsi qu'une perte de l'agrément de conduite ressenti par le conducteur du véhicule.

Selon l'état de la technique, plusieurs solutions permettent la prévention d'un pré-allumage, comme par exemple, l'utilisation de bougies d'allumage spécifiques, l'ajustement du mélange air/carburant ou encore le nettoyage périodique des chambres de combustion. Cependant ces solutions peuvent être complexes à mettre en œuvre. L'état de l'art comporte également des solutions techniques permettant la recirculation de gaz d'échappement refroidis dans la chambre de combustion mais ces technologies présentent un coût élevé et entraînent par ailleurs un apport de matières dans la chambre de combustion, ce qui augmente le risque de pré-allumage dans certaines conditions d'utilisation du moteur.

On connait encore l'article « engine oil development for preventing pre-ignition in turbocharged gasoline engine », SAE International Journal Of Fuels and Lubricants, vol.7, n°3, 15 avril 2014, pp869-874, correspondant au préambule de la revendication 1.

L'invention vise donc à pallier au moins en partie ces inconvénients en proposant un procédé de test simple, fiable et efficace, permettant de réduire les risques de pré-allumage d'un moteur à combustion interne de véhicule.

Plus précisément, pour atteindre cet objectif, la présente invention concerne un procédé de détermination de la propension d'une huile moteur à générer un pré-allumage d'un moteur à combustion interne d'un véhicule, notamment d'un véhicule automobile, ladite huile moteur étant destinée à être introduite dans ledit moteur à combustion interne du véhicule, ledit procédé comprenant :
- une étape de vieillissement de ladite huile moteur pendant un temps de vieillissement prédéterminé,
- après ledit temps de vieillissement prédéterminé, une étape d'évaluation d'une température d'auto-inflammation de l'huile moteur réalisée à haute pression, et
- une étape de comparaison de ladite température d'auto-inflammation de l'huile moteur avec une température prédéterminée, et si la température d'auto-inflammation de l'huile moteur est inférieure à la température prédéterminée cette huile est jugée avoir une propension à générer du préallumage.

Un tel procédé permet avantageusement d'évaluer la température d'auto-inflammation d'une huile moteur en vue d'anticiper le risque de pré-allumage d'un moteur de véhicule, lors de l'utilisation d'une huile moteur vieillie.

De manière avantageuse, le procédé comprend, postérieurement à l'étape de comparaison de la température d'auto-inflammation de l'huile avec la température prédéterminée, une étape d'évaluation d'une distance que le véhicule est en mesure de parcourir, dans des conditions prédéterminées, sans que l'huile moteur ne présente un risque de générer un pré-allumage du moteur du véhicule, en fonction de ladite température d'auto-inflammation. Une telle étape du procédé permet de déterminer la propension de l'huile moteur à générer un pré-allumage du moteur du véhicule, dans le but de s'affranchir avantageusement d'un risque de pré-allumage sur une distance donnée de roulage du véhicule.

Un tel procédé permet de classifier les huiles moteur en vue de prévenir le risque de pré-allumage, permettant d'envisager le développement ultérieur de petits moteurs à essence turbo, particulièrement sujet à la problématique de pré-allumage à vitesse réduite.

Avantageusement, l'étape de vieillissement de l'huile moteur est réalisée pour une pluralité de temps de vieillissement prédéterminés, de préférence 72h et/ou 96h et/ou 120h et/ou 144h, en vue de la réalisation d'une pluralité de test permettant une comparaison en fonction du vieillissement de l'huile moteur.

De manière préférée, le procédé comprend une pluralité d'étapes d'évaluation d'une température d'auto-inflammation, chaque température d'auto-inflammation étant évaluée après un temps de vieillissement prédéterminé différent, permettant la réalisation de gammes pour une huile présentant différentes températures d'auto-inflammation selon le stade de vieillissement de l'huile.

De manière avantageuse, le procédé comprend une pluralité d'étapes d'évaluation d'une pluralité de distances que le véhicule est en mesure de parcourir, dans des conditions prédéterminées, sans que l'huile moteur ne présente un risque de générer un pré-allumage du moteur du véhicule, chaque distance étant évaluée pour un temps de vieillissement prédéterminé différent de l'huile moteur, en fonction de ladite température d'auto-inflammation correspondante. Une telle pluralité d'étapes d'évaluation de distances permet notamment de valider le procédé en confirmant les résultats obtenus.

Avantageusement, le procédé comprend, précédemment à l'étape d'évaluation de la température d'auto-inflammation de l'huile moteur, une étape de mesure d'un temps d'induction à l'oxydation, permettant de déterminer pour une huile donnée le temps nécessaire à l'oxydation de l'huile.

Avantageusement, l'étape d'évaluation de la température d'auto-inflammation de l'huile moteur est réalisée au moyen d'une méthode par calorimétrie différentielle à balayage, permettant l'utilisation d'un calorimètre dont les résultats sont reconnus.

De manière avantageuse, le procédé comprend, précédemment à l'étape d'évaluation de la température d'auto-inflammation de l'huile moteur, une étape d'augmentation de la température de l'huile moteur, de préférence suivant une évolution progressive jusqu'à une température maximale prédéterminée, permettant le déclenchement de l'oxydation de l'huile.

De manière préférée, l'étape d'évaluation de la température d'auto-inflammation de l'huile moteur est réalisée à une haute pression de 10,13 bar, permettant un test standardisé sous haute pression afin de recréer des conditions moyennes similaires aux conditions de pression dans une chambre de combustion, hors combustion.

De manière avantageuse, l'évaluation de la température d'auto-inflammation est réalisée au moyen d'un logiciel informatique dédié à l'analyse de données thermiques, permettant une mesure fiable.

D'autres caractéristiques et avantages de l'invention apparaîtront à la lecture de la description détaillée des modes de réalisation de l'invention, donnés à titre d'exemple uniquement, et en référence aux dessins qui montrent :
- la figure 1, une représentation schématique des étapes d'un procédé de détermination de la propension d'une huile moteur à conduire à un pré-allumage du moteur d'un véhicule, lors de son vieillissement,
- la figure 2, un graphique d'une évolution de la température de l'huile moteur commandée par un calorimètre au cours du procédé selon un mode de réalisation préféré de l'invention, et
- la figure 3, un graphique, permettant l'évaluation de la température d'auto-inflammation d'une huile moteur.

Dans ce qui va suivre, l'invention est décrite en particulier, sans que cela ne puisse être interprété de façon restrictive, en vue de l'évaluation d'une huile destinée à être introduite dans un moteur de véhicule automobile.

Comme décrit précédemment, un véhicule automobile équipé d'un moteur à combustion interne comprend une pluralité de chambres de combustion, dans lesquelles est injecté un mélange d'air et de carburant. La combustion du mélange génère la poussée de pistons montés coulissants dans les chambres de combustion, permettant la mise en rotation de l'arbre moteur et donc des roues du véhicule.

Dans un moteur à essence, la combustion du mélange air/carburant est généralement déclenchée par une bougie présente dans chaque chambre de combustion du moteur et générant une étincelle. Une telle étincelle se produit à un instant précis de manière à optimiser le fonctionnement du moteur.

Cependant, il arrive que le mélange s'auto-enflamme avant la génération de l'étincelle par la bougie. Un tel phénomène, désigné pré-allumage du moteur, apparaît notamment lorsque la vitesse de rotation du moteur est faible, on parle alors de pré-allumage à vitesse réduite ou LPSI, signifiant Low Speed Pre-Ignition en langue anglaise comme décrit précédemment. Un tel pré-allumage à vitesse réduite apparaît notamment lors de l'utilisation d'huiles non stables au vieillissement.

En effet, le moteur comprend un système de lubrification, comprenant de l'huile, permettant de lubrifier différents éléments du moteur afin de limiter leur frottement et leur usure lors de leur mouvement. L'huile circule ainsi notamment au niveau des pistons, du côté extérieur à la chambre de combustion, afin de lubrifier le piston qui se déplace dans le cylindre du moteur. Cependant dans certaines conditions d'utilisation du moteur, de l'huile peut entrer dans la chambre de combustion, entraînant un risque de pré-allumage du moteur à vitesse réduite. En effet, sous l'effet de la chaleur et de l'oxygène, une huile a tendance à s'oxyder, on parle alors de vieillissement de l'huile. Un tel vieillissement d'une huile a pour conséquence la formation de dépôts et donc l'encrassement des chambres de combustion, source de risque de pré-allumage.

L'invention décrite dans ce document propose ainsi de limiter le risque de pré-allumage d'un moteur à vitesse réduite en spécifiant un critère de pré-allumage d'un moteur de véhicule pour une huile vieillie. Pour cela, il va dorénavant être présenté un procédé de détermination de la propension d'une huile à conduire à un pré-allumage du moteur d'un véhicule automobile, lors de son vieillissement.

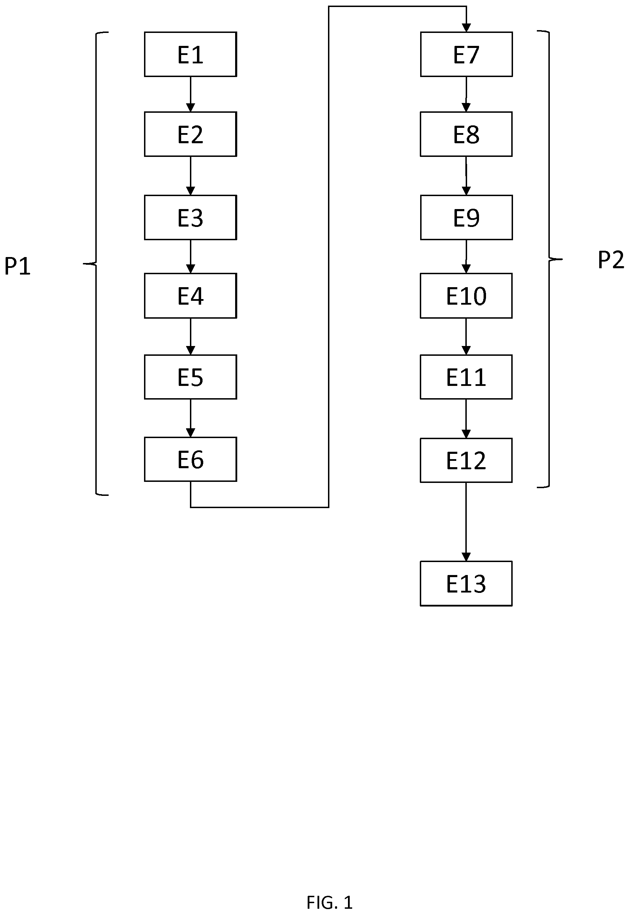

En référence à la figure 1, selon un mode de réalisation préféré, le procédé selon l'invention comprend une étape désignée première phase P1 de vieillissement de l'huile à tester suivie d'une étape désignée deuxième phase P2 de détermination de la propension de l'huile à conduire à un pré-allumage du moteur.

La première phase P1 de vieillissement de l'huile suit de préférence la procédure désignée « Vieillissement par oxydation des huiles moteurs diesel en présence de biocarburant », mise au point par le Groupe Français de Coordination pour le développement des essais de performance des Carburants, des Lubrifiants et autres fluides dans le Transport (référence de la méthode d'essai : GFC Lu-43-A-11 ind2).

La procédure GFC Lu-43-A-11 ind2 décrit un vieillissement par oxydation catalysée, c'est-à-dire par ajout d'une solution de catalyseur à une huile moteur, dans le but de générer une oxydation de l'huile représentative de son vieillissement. Afin de permettre sa mise en œuvre, la première phase P1 du procédé selon l'invention nécessite un récipient, désigné cellule d'essai, et comprend une première étape E1 de préparation d'une telle cellule d'essai. Dans cette étape E1, la cellule d'essai est nettoyée et désinfectée au moyen d'un solvant tel que du perchloréthylène ou éther de pétrole par exemple, puis avec de l'heptane, afin de permettre l'élimination de tout résidu.

Une solution d'essai, contenant l'huile à tester, par exemple 150g d'huile, est ensuite préparée dans une étape E2. Au cours de cette même étape E2, une solution de catalyseur est également préparée. Une telle solution de catalyseur comprend par exemple 1,9023g d'acétylacétonate de Fe(III) anhydre introduit dans une fiole de 100mL, complétée par du chloroforme.

Dans cet exemple, 5mL de la solution de catalyseur sont alors mélangés dans la cellule d'essai aux 150g d'huile, dans le but d'obtenir dans une étape E3 une homogénéisation du mélange huile/catalyseur.

La cellule d'essai comprenant le mélange de la solution d'essai et de la solution de catalyseur est ensuite placée dans un bain chauffant dans une étape E4. Un tube en silicone est alors mis en place afin de raccorder la cellule d'essai à un débitmètre, réglé par exemple à un débit de 10 litres/heure (plus ou moins 0,5 litre/heure), dans le but d'éviter une montée en ébullition du chloroforme pouvant conduire à l'expulsion du bouchon de la cellule d'essai.

Dans cet exemple, le bain chauffant est alors activé dans une étape E5 et programmé à une température de 170°C (plus ou moins 1°C). La température de la cellule d'essai comprenant l'échantillon d'huile à tester est alors régulièrement contrôlée au moyen d'un thermocouple ou d'une thermosonde par exemple. Le bain chauffant est ensuite maintenu à une température de 170°C pendant une durée de 144h par exemple, au terme de laquelle le bain chauffant est stoppé et l'huile prélevée dans une étape E6. Dans cet exemple, trois prélèvements intermédiaires peuvent également être effectués après 72h, 96h et 120h à 170°C.

La première phase P1 du procédé selon l'invention est décrite dans ce document selon un exemple de mode de réalisation, cependant il va de soi que tout variante décrite dans la procédure GFC Lu-43-A-11 ind2 peut être mise en œuvre afin d'aboutir à au moins un échantillon d'huile vieillie. De même, cette procédure de vieillissement peut également être modifiée en incluant une pré-dilution de l'huile, par exemple de 8%, ou bien encore un conditionnement de plusieurs jours à température moyenne, par exemple 30°C.

L'étape E6 de la première phase P1 de vieillissement aboutit ainsi à une pluralité d'échantillons d'huile vieillie, prêts à être utilisés dans la deuxième phase P2 du procédé selon l'invention. Chaque échantillon d'huile HX est par la suite désigné suivant son nombre X d'heures de vieillissement, ainsi une huile HX vieillie au cours de la première phase P1 pendant 144h, sera désignée H144, de même une huile vieillie pendant 96h, sera désignée H96.

La deuxième phase P2 du procédé selon l'invention permet l'évaluation de la température d'auto-inflammation de l'huile HX vieillie au cours de la première phase P1. Pour ce faire, une méthode désignée calorimétrie différentielle à balayage, également connue sous l'acronyme DSC signifiant « differential scanning calorimetry » en langue anglaise, est adaptée et réalisée à haute pression. On entend ici par haute pression une pression représentative de celle pouvant régner dans une chambre de combustion de moteur à combustion interne, en fin de phase de compression, hors combustion. Cette pression est en général supérieure à 1 bar et inférieure à 25 bars.

La calorimétrie différentielle à balayage, habituellement utilisée pour déterminer les transitions thermiques d'un polymère, consiste en une étude de l'évolution et des changements d'états des polymères lorsqu'ils sont chauffés à de hautes températures. En effet, lors d'une calorimétrie différentielle à balayage, deux cellules d'essai sont placées dans un calorimètre, l'une contient un échantillon à tester et la seconde est vide. Lors d'une montée en température du calorimètre, l'échantillon à tester emmagasine de la chaleur, la cellule comprenant l'échantillon nécessite ainsi une énergie supplémentaire pour chauffer à la même température que la cellule vide. Cette quantité de chaleur supplémentaire est mesurée par le calorimètre au moyen de thermocouples ou de thermosondes par exemple relié(e)s aux cellules.

Un tel procédé est réalisé dans des conditions dites inertes, c'est-à-dire que les cellules introduites dans le calorimètre sont purgées par exemple au moyen de nitrogène. Lors de la montée en température, une réaction chimique se produit, transformant le gaz nitrogène en oxygène. Une telle réaction se produit pendant un temps, désigné temps d'induction à l'oxydation (TIO) et correspondant au temps nécessaire entre le changement de gaz et le début de l'oxydation. Lorsque le temps d'induction à l'oxydation est élevé, on dit que le matériau présente une bonne stabilité à l'oxydation.

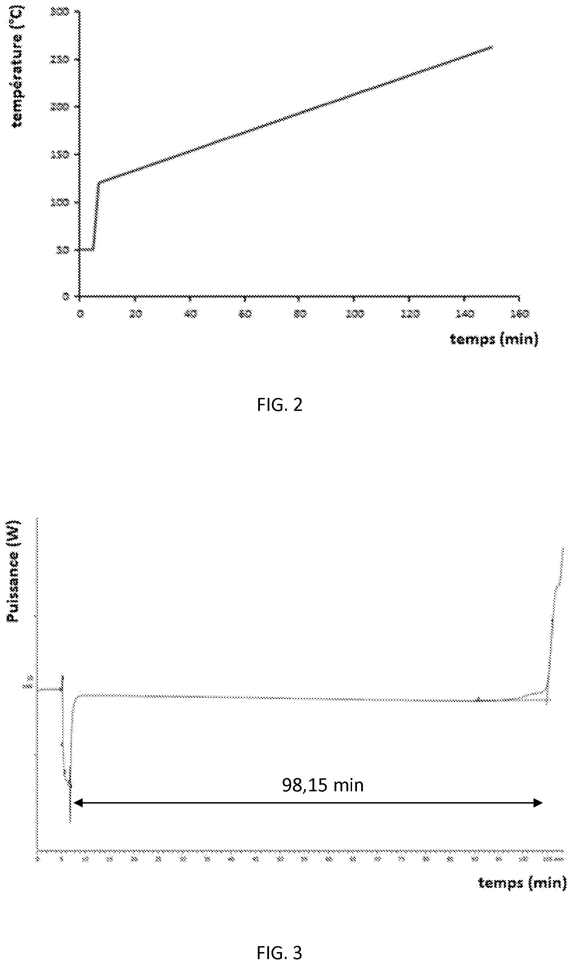

Une telle mesure du temps d'induction à l'oxydation permet selon un mode de réalisation de l'invention d'évaluer la température d'auto-inflammation d'une huile vieillie à tester. La température d'auto-inflammation est évaluée sur une courbe, telle que montrée à la figure 3, par la règle de la tangente généralement appliquée lors d'analyses de calorimétrie différentielle à balayage et couramment mise en œuvre par l'homme du métier.

Ainsi, dans le but d'étudier une huile HX vieillie, le procédé nécessite un appareil de calorimétrie différentielle à balayage ainsi que l'utilisation de deux cellules d'essai. La deuxième phase P2 du procédé selon l'invention comprend ainsi une étape E7 de nettoyage des cellules au moyen d'un ou plusieurs solvant(s), de préférence le n-Heptane et l'acétone.

Dans cet exemple, deux cellules sont nécessaires à la mise en œuvre du procédé comme décrit précédemment, une première cellule est conservée vide et une seconde cellule est remplie d'huile HX vieillie, par exemple 2 milligrammes (plus ou moins 0,05 mg), dans une étape E8. Les deux cellules sont alors placées dans le calorimètre, qui est alors scellé. Du nitrogène est ensuite introduit afin de créer les conditions inertes décrites précédemment. Le calorimètre est alors configuré afin de commander dans une étape E9 la montée en température des deux cellules. Une pression, par exemple de 10,13 bar, est également appliquée, afin de reproduire les conditions de pression à l'intérieur d'une chambre de combustion de moteur à combustion interne de véhicule automobile, hors combustion.

En référence à la figure 2, la montée en température à l'intérieur du calorimètre évolue de manière progressive. Dans cet exemple, le calorimètre commande une évolution de la température rapide jusqu'à un premier palier de 50°C, auquel la température est maintenue pendant 5 minutes. La température augmente ensuite progressivement jusqu'à un second palier, par exemple de 40°C par minute jusqu'à 120°C. L'évolution est ensuite commandée plus progressivement, par exemple 1°C par minute jusqu'à atteindre une température de 250°C.

Une telle température élevée permet le changement du nitrogène en oxygène, permettant ainsi la mesure du temps d'induction à l'oxydation, dans une étape E10. Le temps d'induction à l'oxydation est mesuré à l'apparition de l'oxydation de préférence au moyen d'un logiciel informatique relié au calorimètre, par exemple STARe-Software.

Une fois le temps d'induction à l'oxydation de la cellule comprenant l'huile vieillie à tester mesuré, le logiciel permet de déterminer l'énergie produite par la cellule dans le but d'atteindre la température de 250°C demandée. Le logiciel permet alors, dans une étape E11, d'évaluer la température d'auto-inflammation sous pression d'une telle huile. La figure 3 illustre un graphique tracé par le logiciel STARe-Software représentant l'évolution de l'énergie produite afin que l'huile atteigne la température souhaitée en fonction du temps et présentant l'évaluation de la température d'auto-inflammation de l'huile par la méthode de la tangente.

La température d'auto-inflammation d'une huile en fonction de son vieillissement permet de déterminer la propension de l'huile à conduire à un pré-allumage du moteur. La température d'auto-inflammation de l'huile moteur est inférieure à la température prédéterminée cette huile est jugée avoir une propension à générer du préallumage. En effet, la température d'auto-inflammation de l'huile est comparée, dans une étape E12, à une température prédéterminée, par exemple 205°C. Une telle comparaison permet enfin d'évaluer, dans une étape E13, la distance qu'un véhicule peut parcourir, dans un pays à température tempérée et avec un carburant n'impactant pas la stabilité de l'huile, avant que l'huile ne présente un risque de pré-allumage du moteur, en fonction de la température d'auto-inflammation correspondante évaluée à l'étape E11.

Dans cet exemple, une huile peut être testée sous plusieurs conditions de vieillissement comme décrit précédemment. Ainsi le procédé décrit dans ce document peut être répété pour une même huile avec une pluralité d'échantillons de cette huile prélevés à différents stades de vieillissement, par exemple au bout de 72h, 96h ou 120h de vieillissement.

A titre d'exemple, après 144h de vieillissement, si la température d'auto-inflammation est supérieure à 205°C, alors l'huile H144 est considérée comme n'étant pas susceptible de générer un pré-allumage à vitesse réduite pendant 30 000 km dans un pays à température tempérée et avec un carburant non impactant sa stabilité par exemple. De façon similaire, après 72h de vieillissement, si la température d'auto-inflammation est supérieure à 205°C, alors l'huile H72 est considérée comme n'étant pas susceptible de générer un pré-allumage à vitesse réduite pendant 10 000 km dans un pays à température tempérée et avec un carburant non impactant sa stabilité par exemple.

Un tel procédé de détermination de la propension d'une huile à conduire à un pré-allumage du moteur permet ainsi de créer des gammes d'huiles destinées à être introduites dans un système de lubrification de moteur. De telles gammes permettent ainsi avantageusement de classer les huiles en fonction de leur propension à générer un pré-allumage suivant leur température d'auto-inflammation.

## Revendications

1. Procédé de détermination de la propension d'une huile moteur à générer un pré-allumage d'un moteur à combustion interne d'un véhicule, notamment d'un véhicule automobile, ladite huile moteur étant destinée à être introduite dans ledit moteur à combustion interne du véhicule, ledit procédé comprenant une étape (P1) de vieillissement de ladite huile moteur pendant un temps de vieillissement prédéterminé, **caractérisé en ce qu'**il comprend de plus :
- après ledit temps de vieillissement prédéterminé, une étape (E11) d'évaluation d'une température d'auto-inflammation de l'huile moteur réalisée à haute pression,
- une étape (E12) de comparaison de ladite température d'auto-inflammation de l'huile moteur avec une température prédéterminée, et si la température d'auto-inflammation de l'huile moteur est inférieure à la température prédéterminée cette huile est jugée avoir une propension à générer du préallumage.

2. Procédé selon la revendication précédente, comprenant, postérieurement à l'étape (E12) de comparaison de la température d'auto-inflammation de l'huile avec la température prédéterminée, une étape (E13) d'évaluation d'une distance que le véhicule est en mesure de parcourir, dans des conditions prédéterminées, sans que l'huile moteur ne présente un risque de générer un pré-allumage du moteur du véhicule, en fonction de ladite température d'auto-inflammation.

3. Procédé selon l'une des revendications précédentes, dans lequel l'étape (P1) de vieillissement de l'huile moteur est réalisée pour une pluralité de temps de vieillissement prédéterminés.

4. Procédé selon la revendication précédente, comprenant une pluralité d'étapes (E11) d'évaluation d'une température d'auto-inflammation, chaque température d'auto-inflammation étant évaluée après un temps de vieillissement prédéterminé différent.

5. Procédé selon la revendication précédente, comprenant une pluralité d'étapes (E13) d'évaluation d'une pluralité de distances que le véhicule est en mesure de parcourir, dans des conditions prédéterminées, sans que l'huile moteur ne présente un risque de générer un pré-allumage du moteur du véhicule, chaque distance étant évaluée pour un temps de vieillissement prédéterminé différent de l'huile moteur, en fonction de la température d'auto-inflammation correspondante.

6. Procédé selon l'une des revendications précédentes, comprenant, précédemment à l'étape (E11) d'évaluation de la température d'auto-inflammation de l'huile moteur, une étape (E10) de mesure d'un temps d'induction à l'oxydation de l'huile.

7. Procédé selon l'une des revendications précédentes, dans lequel l'évaluation de la température d'auto-inflammation de l'huile moteur est réalisée au moyen d'une méthode par calorimétrie différentielle à balayage.

8. Procédé selon l'une des revendications précédentes, comprenant, précédemment à l'étape (E11) d'évaluation de la température d'auto-inflammation de l'huile moteur, une étape (E9) d'augmentation de la température de l'huile moteur, de préférence suivant une évolution progressive jusqu'à une température maximale prédéterminée.

9. Procédé selon l'une des revendications précédentes, dans lequel l'évaluation de la température d'auto-inflammation de l'huile moteur est réalisée à une haute pression de 10,13 bar.

10. Procédé selon l'une des revendications précédentes, dans lequel l'évaluation de la température d'auto-inflammation est réalisée au moyen d'un logiciel informatique dédié à l'analyse de données thermiques.

## Patentansprüche

1. Ein Verfahren zur Bestimmung der Neigung von einem Motoröl zu erzeugen, eine Vorzündung einer Brennkraftmaschine eines Fahrzeugs, insbesondere eines Kraftfahrzeuges, dem Motoröl ist dazu bestimmt werden, eingeführt in die Motorverbrennungsinnen von Fahrzeugs, wob ei Verfahren, umfassend einen Schritt (P1) Alter der Ölmotor für eine vorgegebene Alterungszeit,
**dadurch gekennzeichnet, dass** es ferner umfasst:
- nach der vorbestimmten Alterungszeit, ein Schritt (E11) zur Auswertung einer Selbstentzündungstemperatur des Motoröls durchgeführt bei einem hohen Druck,
- einen Schritt (E12), in dem die Selbstentzündungstemperatur des Motoröls mit einer vorbestimmten Temperatur verglichen wird und die Temperatur des Selbstentzündungsölmotors niedriger ist als die vorbestimmte Temperatur, bei der das Öl eine Neigung zur Vorerzeugung aufweist -Zündung.

2. Verfahren nach dem vorhergehenden Anspruch, umfassend, nach dem Schritt (E12) der SELF Vergleich -Zündung Temperatur des Öls mit der vorbestimmten Temperatur, ein Schritt (E13) ein Abstands Auswertung, dass das Fahrzeug in der Lage, zu reisen, unter vorbestimmten Bedingungen, ohne das Motoröl zu präsentieren ein Risiko der Erzeugung eines Vorzündung des Fahrzeugmotors als Funktion der Selbstentzündungstemperatur.

3. Verfahren nach einem der vorhergehenden Ansprüche, bei dem der Schritt (P1) des Alterns des Motoröls für mehrere vorbestimmte Alterungszeiten durchgeführt wird.

4. Verfahren nach dem vorhergehenden Anspruch, umfassend mehrere Schritte (E11) zum Bewerten einer Selbstentzündungstemperatur, wobei jede Selbstentzündungstemperatur nach einer anderen vorbestimmten Alterungszeit bewertet wird.

5. Verfahren nach dem vorhergehenden Anspruch, umfassend mehrere Schritte (E13) zum Bewerten einer Vielzahl von Entfernungen, die das Fahrzeug unter vorbestimmten Bedingungen zurücklegen kann, ohne dass das Motoröl ein Risiko darstellt, eine Vorzündung des zu erzeugen Motor des Fahrzeugs, wobei jeder Abstand für eine vorbestimmte Alterungszeit, die sich vom Motoröl unterscheidet, als Funktion der entsprechenden Selbstentzündungstemperatur bewertet wird.

6. Verfahren nach einem der vorhergehenden Ansprüche, umfassend, zuvor in Schritt (E11) der Auswerteschaltung Selbstentzündungstemperatur des Motoröls, einen Schritt (E10) einer Induktionszeit der Oxidation Messung von Öl.

7. Verfahren nach einem der vorangehenden Ansprüche, bei dem die Bewertung der Selbstentzündungstemperatur des Motoröls mittels eines Differentialscanningkalorimetrieverfahrens durchgeführt wird.

8. Verfahren nach einem der vorhergehenden Ansprüche, umfassend, zuvor in Schritt (E11) das Bewerten der Selbstentzündungstemperatur des Motoröls, einen Schritt (E9) zum Erhöhen der Temperatur des Motoröls, Motoröl, vorzugsweise nach einem allmählichen Entwicklung bis zu einer vorgegebenen Maximaltemperatur.

9. Verfahren nach einem der vorhergehenden Ansprüche, bei dem die Bewertung der Selbstentzündungstemperatur des Motoröls bei einem hohen Druck von 10, 13 bar durchgeführt wird.

10. Verfahren nach einem der vorhergehenden Ansprüche, bei dem die Bewertung der Selbstentzündungstemperatur mittels einer Computersoftware durchgeführt wird, die der Analyse von thermischen Daten gewidmet ist.

## Claims

1. A method for determining the propensity of an engine oil to generate a pre-ignition of an internal combustion engine of a vehicle, in particular a motor vehicle, said engine
oil is intended to be introduced into the said engine combustion internal of u vehicle, said method comprising a step (P1) aging said oil motor for a predetermined aging time,
**characterized in that** it further comprises :
- after said predetermined aging time, a step (E11) for evaluating an auto-ignition temperature of the engine oil carried out at high pressure,
- a step (E12) comparing said temperature of self-ignition of the engine oil with a predetermined temperature, and if the temperature of self-ignition oil engine is lower than the predetermined temperature the oil is deemed to have a propensity to generate pre-ignition.

2. Method according to the preceding claim, comprising, after step (E12) of comparing the self -ignition temperature of the oil with the predetermined temperature, a step (E13) of evaluating a distance that the vehicle is able to travel, under predetermined conditions, without the engine oil presenting a risk of generating a pre-ignition of the vehicle engine, as a function of said auto-ignition temperature.

3. Method according to one of the preceding claims, in which the step (P1) of aging the engine oil is carried out for a plurality of predetermined aging times.

4. Method according to the preceding claim, comprising a plurality of steps (E11) for evaluating an auto-ignition temperature, each auto-ignition temperature being evaluated after a different predetermined aging time.

5. Method according to the preceding claim, comprising a plurality of steps (E13) for evaluating a plurality of distances that the vehicle is able to cover, under predetermined conditions, without the engine oil presenting a risk to generate a pre-ignition of the engine of the vehicle, each distance being evaluated for a predetermined aging time different from the engine oil, as a function of the corresponding auto-ignition temperature.

6. Method according to one of the preceding claims, comprising, previously in step (E11) of evaluating the auto-ignition temperature of the engine oil, a step (E10) of measuring an induction time to the oxidation of oil.

7. Method according to one of the preceding claims, in which the evaluation of the auto-ignition temperature of the engine oil is carried out by means of a differential scanning calorimetry method.

8. Method according to one of the preceding claims, comprising, previously in step (E11) of evaluating the auto-ignition temperature of the engine oil, a step (E9) of increasing the temperature of the engine oil. engine oil, preferably following a gradual evolution up to a predetermined maximum temperature.

9. Method according to one of the preceding claims, in which the evaluation of the auto-ignition temperature of the engine oil is carried out at a high pressure of 10, 13 bar.

10. Method according to one of the preceding claims, in which the evaluation of the auto-ignition temperature is carried out by means of computer software dedicated to the analysis of thermal data.
